# EUROPEAN PATENT SPECIFICATION

(11) **EP 2 675 487 B1**
(45) Date of publication and mention of the grant of the patent: **17.10.2018**
(21) Application number: 12755058.0
(22) Date of filing: 09.02.2012
(51) Int. Cl.: A61K 51/04, A61K 51/08, A61K 51/06, C07F 9/09, C07F 9/10

(54) **NON-INVASIVE IN VIVO METHODS OF MONITORING BIODISTRIBUTION WITH RADIOLABELED COMPOUNDS**
NICHT-INVASIVE IN-VIVO-VERFAHREN ZUR ÜBERWACHUNG EINER BIOVERTEILUNG MIT RADIOAKTIV MARKIERTEN VERBINDUNGEN
PROCÉDÉS IN VIVO NON-INVASIFS DE SURVEILLANCE DE BIODISTRIBUTION AVEC DES COMPOSÉS RADIOMARQUÉS

(30) Priority: 09.02.2011 NZ 59104711
(43) Date of publication of application: 25.12.2013
(73) Proprietor: Bovin, Nicolai Vladimirovich, Moscow 117437 (RU); Henry, Stephen Micheal, Auckland (NZ); Korchagina, Elena Yurievna, Moscow 117218 (RU); Rodionov, Igor Leonidovich, Moskovskaya obl., Pushchino142292 (RU); Tuzikov, Alexander Borisovich, Moscow 105037 (RU)
(72) Inventor: Bovin, Nicolai Vladimirovich, Moscow 117437 (RU); Henry, Stephen Micheal, Auckland (NZ); Korchagina, Elena Yurievna, Moscow 117218 (RU); Rodionov, Igor Leonidovich, Moskovskaya obl., Pushchino142292 (RU); Tuzikov, Alexander Borisovich, Moscow 105037 (RU)
(74) Representative: Gowshall, Jonathan Vallance
(86) International application number: PCT/NZ2012/000012
(87) International publication number: WO 2012/121610

(56) References cited:
- EP-A1- 0 094 251
- EP-A1- 0 588 754
- WO-A1-2009/048343
- WO-A1-2009/048343
- WO-A2-2008/030115
- US-A- 4 571 332
- ELIZABETH M HADAC ET AL: "Fluorescein and radiolabeled Function-Spacer-Lipid constructs allow for simpleandbioimaging of enveloped virions", JOURNAL OF VIROLOGICAL METHODS, ELSEVIER BV, NL, vol. 176, no. 1, 7 June 2011 (2011-06-07), pages 78-84, XP028243695, ISSN: 0166-0934, DOI: 10.1016/J.JVIROMET.2011.06.005 [retrieved on 2011-06-14]
- TSOMIDES T J ET AL: "Stoichiometric Labeling of Peptides by Iodination on Tyrosyl or Histidyl Residues", ANALYTICAL BIOCHEMISTRY, ACADEMIC PRESS INC, NEW YORK, vol. 210, no. 1, 1 April 1993 (1993-04-01) , pages 129-135, XP024763924, ISSN: 0003-2697, DOI: 10.1006/ABIO.1993.1162 [retrieved on 1993-04-01]
- D. BLAKE ET ALL: "Fluorophore-kodecytes - fluorescent function-spacer-lipid (FSL) modified cells for in vitro and in vivo analyses", FEBS JOURNAL, vol. 277, no. (Suppl 1), C3.12, 2010, pages 199-199, XP002756080,
- MASATO YAMAMOTO ET AL: "Current Issues and Future Directions of Oncolytic Adenoviruses", MOLECULAR THERAPY, vol. 18, no. 2, 24 November 2009 (2009-11-24), pages 243-250, XP055262339, GB ISSN: 1525-0016, DOI: 10.1038/mt.2009.266
- HADAC, E. M. ET AL.: 'Fluorescein and radiolabeled Function-Spacer-Lipid constructs allow for simple in vitro and in vivo bioimaging of enveloped virions' JOURNAL OF VIROLOGICAL METHODS vol. 176, 2011, pages 78 - 84, XP028243695

## Description

### FIELD OF INVENTION

The invention relates to a non-invasive method of monitoring the distribution of cells and virions *in vivo* and constructs for use in the method.

### BACKGROUND ART

Existing methods for monitoring the distribution of biological entities *in vivo* require covalent or genetic modification of the entity. Such modification may result in the behaviour of the biological entity *in vivo* being modified.

Therapeutic applications where viruses are administered to a subject are in development. These applications include gene therapy and tumour destruction (oncolytic virotherapy) (Lui *et al* (2007)). In the development of these therapies it is a requirement to be able to monitor the distribution of the virion following administration without altering its infectivity (Herschman (2003); Yamamoto and Curiel (2010))

Monitoring the distribution of virions following administration is problematic employing existing methods. Tissue biopsy is invasive and is of limited use in clinical trials (Kuruppu and Tanabe (2005); Waehler *et al* (2007)). Non-invasive methods such as magnetic resonance imaging (MRI), positron emission tomography (PET) and X-ray computed tomography (CT) usually require image enhancing agents or chemical modification of the virion (Piwnica-Worms *et al* (2004); Strable and Finn (2009)).

Viruses may be engineered to incorporate reporter genes. However, monitoring the expression of these genes only indicates where the genes of the virus are being expressed (Peng *et al* (2003); Waehler *et al* (2007)).

EP 0094251 A1 discloses 4- or 6- substituted aldosterones, their production and use in immunoassay. WO 2009/048343 A1 discloses methods for effecting qualitative and quantitative changes in the functional moieties expressed at the surface of cells and multicellular structures, and functional lipid constructs for use in such methods.

It is an object of this invention to provide a convenient alternative to these existing methods with application to biological entities in addition to enveloped viruses (virions).

### STATEMENT OF INVENTION

The invention is defined in the appended claims. Any subject-matter falling outside the scope of the claims is provided for information purposes only. In a **first** aspect the disclosure provides a method of radiolabelling a biological entity comprising the step of contacting a suspension of the biological entity with an iodinated (¹²⁵I) construct of the structure F-S-L for a time and at a temperature sufficient to allow localisation of the ¹²⁵I to the surface of the biological entity where F comprises an iodinated histidine (His) residue or an iodinated tyrosine (Tyr) residue, S is a spacer covalently linking F to L, and L is a lipid.

In a **second** aspect the disclosure provides a non-invasive method of monitoring the distribution of a biological entity in a subject *in vivo* comprising the steps of:
- contacting a suspension of the biological entity with a an iodinated (¹²⁵I) construct of the structure F-S-L for a time and at a temperature sufficient to provide a ¹²⁵I-labelled biological entity;
- administering to the subject the ¹²⁵I-labelled biological entity to a subject; and
- monitoring the distribution of the biological entity by radioactivity bioscanning,
where F comprises an iodinated histidine (His) residue or an iodinated tyrosine (Tyr) residue, S is a spacer covalently linking F to L, and L is a lipid.

Preferably, the suspension is an aqueous suspension. S is selected to provide a construct of the structure F-S-L that is readily dispersible in water yet spontaneously incorporates into lipid bilayers, including the membranes of cells and enveloped viruses. Preferably, the biological entity is a cell or an enveloped virus. The ¹²⁵I-labelled biological entity may be administered to the subject by injection. Preferably, the ¹²⁵I-labelled biological entity is administered to the subject by intraperitoneal infusion or intravenous injection.

Preferably, F comprises an iodinated tyrosine (Tyr) residue. More, preferably the tyrosine (Tyr) residue is an N-carbonyl-Tyr residue. Most preferably, the tyrosine (Tyr) residue is an N-carbonyl-Tyr residue selected from the group consisting of: N-COH (*N*-formyl) Tyr residues and *N*-CO(CH₂)₂COOH Tyr residues.

In a **third** aspect the disclosure provides constructs of the structure F-S-L for use in the methods of the first aspect and the second aspect of the disclosure.

In an embodiment of the invention, the constructs are of the structure: 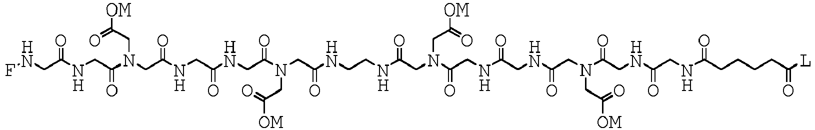 where M is H or CH₃.

According to the invention, F is a tyrosine residue selected from the group consisting of: 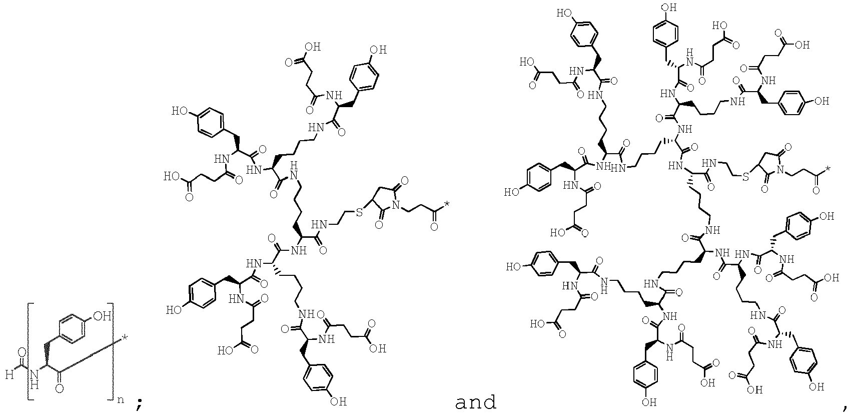 where n is an integer from 1 to 6 inclusive.

According to the invention, L is phosphatidylethanolamine. More preferably, L is is selected from the group consisting of: 1,2-0-dioleoyl-sn-glycero-3-phosphatidylethanolamine (DOPE) and 1,2-0-distearyl-sn-glycero-3-phosphatidylethanolamine (DSPE). Most preferably, L is 1,2-0-dioleoyl-sn-glycero-3-phosphatidylethanolamine (DOPE).

In a first embodiment of the third aspect, the constructs of the invention are of the structure *N*-formyl-(Tyr)ₙ-S-L where n is an integer from 1 to 6 inclusive. Preferably, n is 1 and the construct is of the structure: 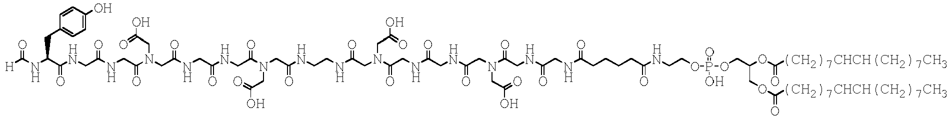 designated FSL-tyrosine.

In a second embodiment of the third aspect, the constructs of the invention are of the structure: 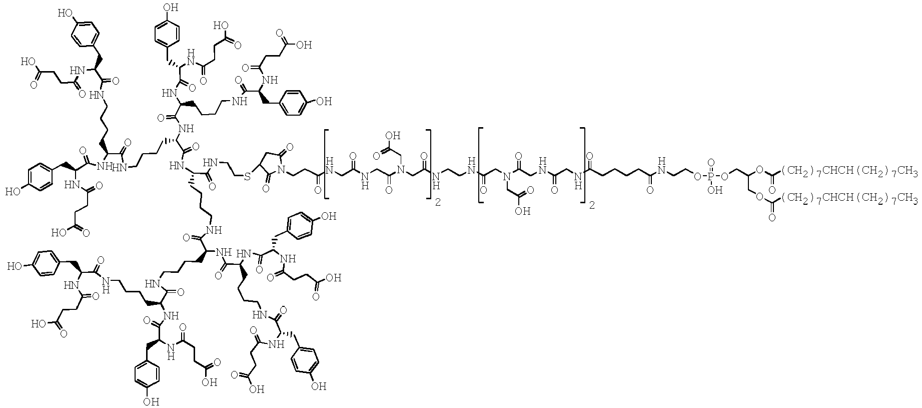 designated FSL-Tyr8.

In a second embodiment of the third aspect, the constructs of the invention are of the structure: 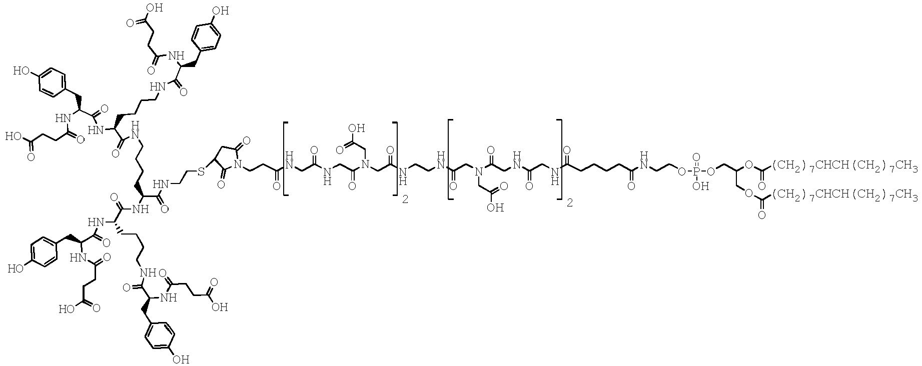 designated FSL-Tyr4.

In the description and claims of this specification the following acronyms, terms and phrases have the meaning provided:
"Aqueous suspension" means a suspension prepared in water with or without the addition of buffers or salts and specifically includes a suspension in saline.
"Biological entity" means a cell, multicellular structure or virus.
"Localised" means associated with a surface by non-covalent interactions and "localising" and "localisation" have a corresponding meaning.
"Monovalent cation (M)" means a counter ion having a positive charge of one (+1).
"Readily dispersible in water" means forms a stable, monophasic dispersion on contact with water at 25 °C in the absence of detergents or organic solvents.

"( )^{x}", "( )ₓ", "[ ]ₓ" and "[ ]^{x}" mean the group contained in the parentheses is repeated a number (x) of times. By way of illustration: 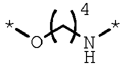 means the methylene group (-CH₂-) is repeated 4 times and the structure represented is equivalent to:

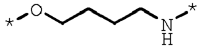

The terms "first", "second", "third", etc. used with reference to elements, features or integers of the subject matter defined in the Statement of Invention and Claims, or when used with reference to alternative embodiments of the invention are not intended to imply an order of preference.

The invention will now be described with reference to embodiments or examples and the figures of the accompanying drawings pages.

### BRIEF DESCRIPTION OF DRAWINGS

**Figure 1****.** Autoradiograph of free Na¹²⁵I (left lane) and iodinated (¹²⁵I) FSL-tyrosine (right lane) run on 15% acrylamide gel SDS-PAGE in nonreducing sample buffer.
**Figure 2****.** Iodinated (¹²⁵I) FSL-tyrosine insertion into Vero cells. Vero cells 5 x 10⁵ cells/well were contacted with either iodinated (¹²⁵I) FSL-tyrosine or ¹²⁵I (of equal cpm) and incubated for 2 hours at 37 °C. Washed cells were lysed then radioactivity measured.

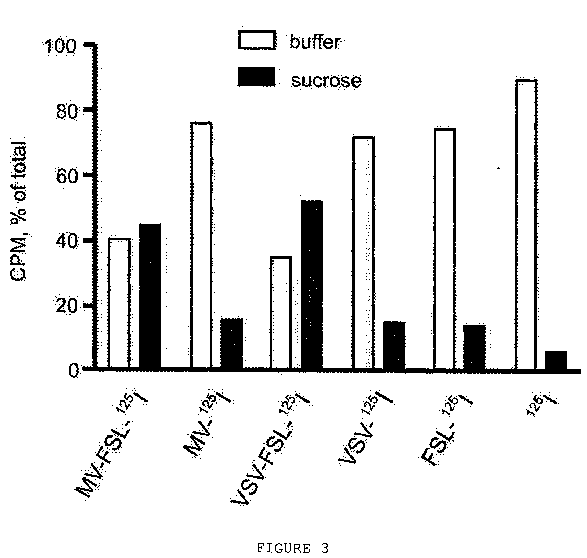

**Figure 3****.** VSV and MV incubated for 1 hour at room temperature with iodinated (¹²⁵I) FSL-tyrosine or Na¹²⁵I and harvested from the buffer or 20% sucrose portion of a step gradient tube following centrifugation for 75 min at 200g.
**Figure 4****.** Ultracentrifuge 20% sucrose fractionations of iodinated (¹²⁵I) FSL-tyrosine or ¹²⁵I labeled VSV and MV.

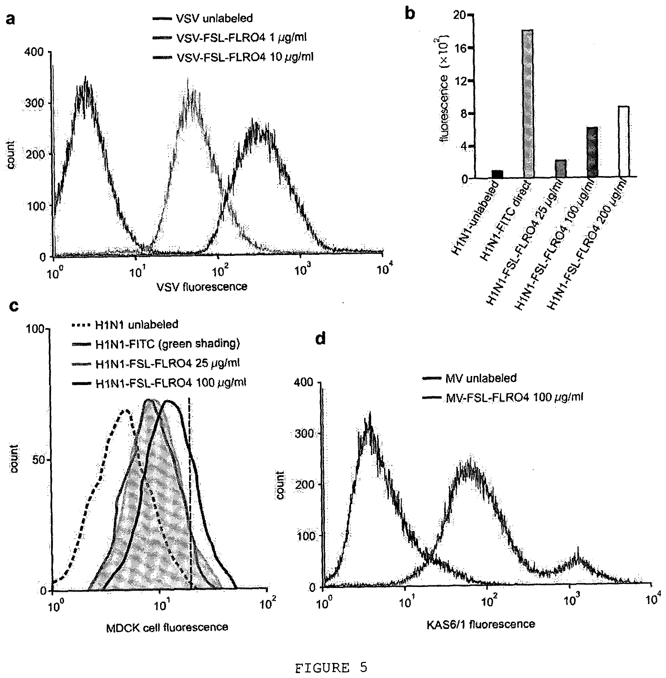

**Figure 5****.** FSL-fluorescein (FSL-FLRO4) labelling of virions. (a) FACScan of FSL-fluorescein modified VSV. (b) Total fluorescence of H1N1 samples (8 haemagglutinin units of virus was taken in all cases) and labelled directly with FITC or modified with different concentrations of FSL-fluorescein(FSL-FLRO4). The fluorescence was read in 96-well plate with Victor2 multilabel counter (PerkinElmer, USA) at 495 nm. (c) FACScan of MDCK cells (transfected with 6-sialyltransferase) and infected with human A/Puerto Rico/8/1934 (H1N1) labelled either directly with FITC (green) or increasing levels of FSL-fluorescein (FSL-FLRO4). (d) FACScan of virions attached to a KAS6/1 cell suspension labelled with FSL-fluorescein (FSL-FLRO4) modified MV.
**Figure 6****.** Biodistribution of intraperitoneal (IP) infusions of iodinated (¹²⁵I) FSL-tyrosine (FSL-¹²⁵I) modified VSV, iodinated (¹²⁵I) FSL-tyrosine (FSL-¹²⁵I) and ¹²⁵I. (a) Scans of two animals 1 hour post IP infusion and a sequential scan of one of the two animals at 4 and 24 hours post IP infusion. (b) Distribution by organ/tissue at one hour post IP infusion. (c) Distribution by organ/tissue at two hours post IP infusion.

### DETAILED DESCRIPTION

The invention is defined in the appended claims. The invention provides a convenient method of radio-labelling biological entities. The constructs of the invention are dispersible in water, spontaneously incorporate into membranes and conveniently iodinated (¹²⁵I) . The localisation of ¹²⁵I to the biological entity by use of the iodinated construct permits real time monitoring of the distribution of the biological entity *in vivo* by non-invasive detection methods.

Known methods of radioiodination employ direct labelling of proteins or other target molecules or indirect labelling by first labelling an intermediate compound which is then used to perform the final modification (Hermanson (2008)). These known methods require termination of the direct labelling step or control of the final modification to ensure the correct degree of iodination. The constructs of the invention may be prepared with predetermined levels of radioactivity and used to quantitatively iodinate the biological entity. Use of constructs (F-S-L) comprising oligomers of tyrosine (FSL-Tyrosine) or dendrimers of tyrosine (FSL-Tyr4 and FSL-Tyr8) as the tyrosine (Tyr) residue (F) permits radiolabelling of biological entities at a range of specific activities with no variation in the degree of labelling of proteins or other target molecules.

Water dispersible constructs have been used both *in vitro* and *in vivo* to modify the surface of cells (Frame *et al* (2007); Heathcote *et al* (2010); Oliver *et al* (2011); Henry (2009)). The *in vivo* survival of red blood cells modified in this way has been demonstrated (Oliver *et al* (2011)). The normal *in vivo* development of murine embryos modified in this way has also been demonstrated (Blake *et al* (2003)).

The surface of enveloped virions is hydrophobic. The localisation of a fluorophore to the surface of virions has now been demonstrated *in vitro* using the construct designated FSL-fluorescein (Blake *et al* (2010)). A number of virions have been modified to incorporate this construct; vesicular stomatitis virus human IFNβ virus (VSV), measles virus encoding the human thyroidal sodium iodide symporter (MV) and H1N1 influenza virus (H1N1). The novel construct designated FSL-tyrosine can be readily iodinated (¹²⁵I) and used in an analogous way to permit the real time monitoring of the distribution of virions *in vivo.*

The modification of virions using the dispersible constructs of the invention is dose dependent. The method also avoids exposure of the virions to incompatible reagents. The modification does not appear to significantly affect the infectivity of the virions or their ability to bind cell membranes as demonstrated by H1N1 fusion with ST cells and MV binding to KAS6/1 cells.

The distribution of VSV modified by the iodinated (¹²⁵I) construct FSL-tyrosine is consistent with previously reported observations (Peng *et al* (2003)). Within an hour of administration the radiolabel localized to liver, spleen and bloodstream. Using the thyroid signal as an indicator it appears the modified virions are maintained *in vivo* for at least 4 hours. The localisation of modified virions to specific tissues can therefore be monitored quantitatively.

### MATERIALS AND METHODS

Trifluoroacetic acid (TFA), trifluoroethanol (TFE), 4-dimethylaminopyridine (DMAP), di-tert-Butyldicarbonate (Boc₂O), 1-hydroxybenzotriazole (HOBt), DIEA - diisopropylethylamine, 0-(benzotriazol-1-yl)-N,N,N' ,N'-tetramethyluronium tetrafluoroborate(TBTU), Boc-Lys(Boc)-OH*DCHA and cysteamine were obtained from IRIS Gmbh (Germany). *p*-Nitrophenyl ester of N-ter-butyloxycarbonyl-tyrosine benzyl ether (Boc-Tyr(Bzl)-ONp) was obtained from Reanal (Hungary). Succinic anhydride and HF gas were obtained from Fluka.

Vesicular stomatitis virus human IFND virus (VSV), and Measles virus MV-NIS (MV) were supplied by the Viral Vector production laboratory, Mayo Clinic. Influenza virus (H1N1) A/Puerto Rico/8/1934 (H1N1) human viruses were supplied by Institute of Virology RAS, Moscow. All native and labeled viruses were purified by ultracentrifugation in 20% sucrose. Age-matched (4-6 weeks old) female C57Bl mice (Harlan Sprague Dawley, Indianapolis, IN) were housed in a specific pathogen-free facility. All animal studies were approved by, and performed according to guidelines of a bioethics committee.

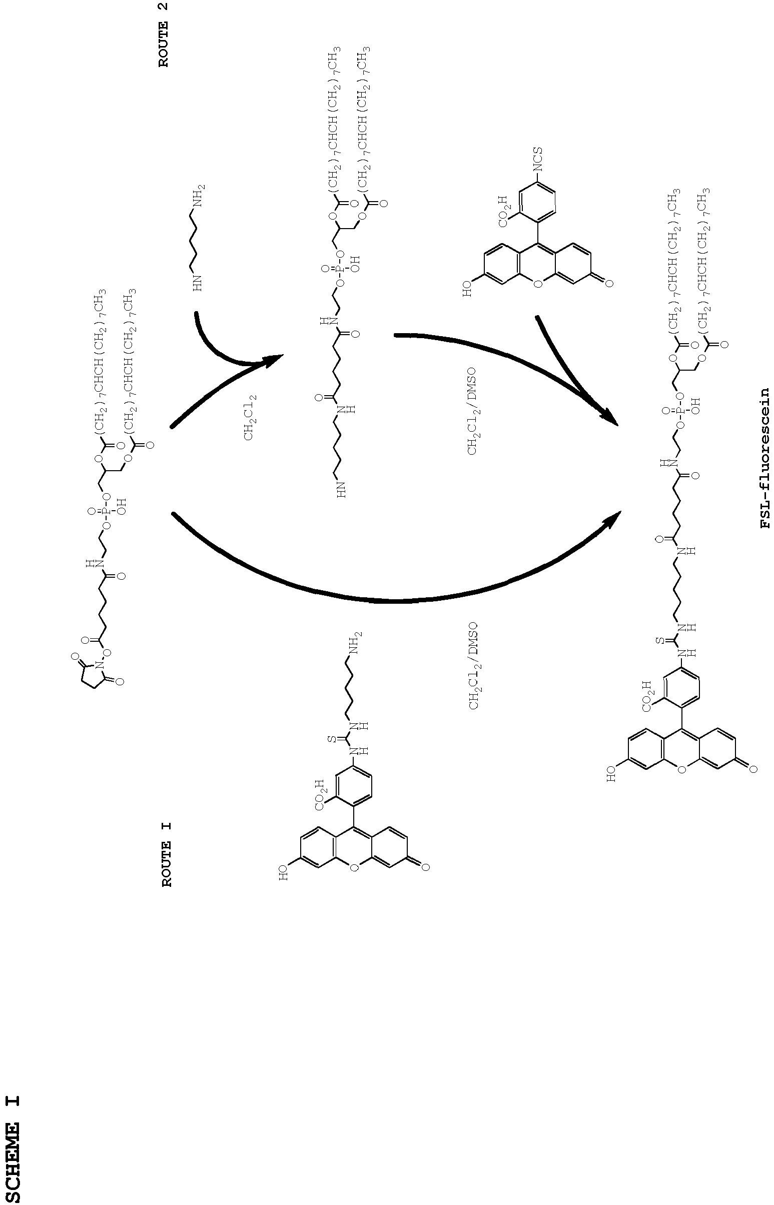

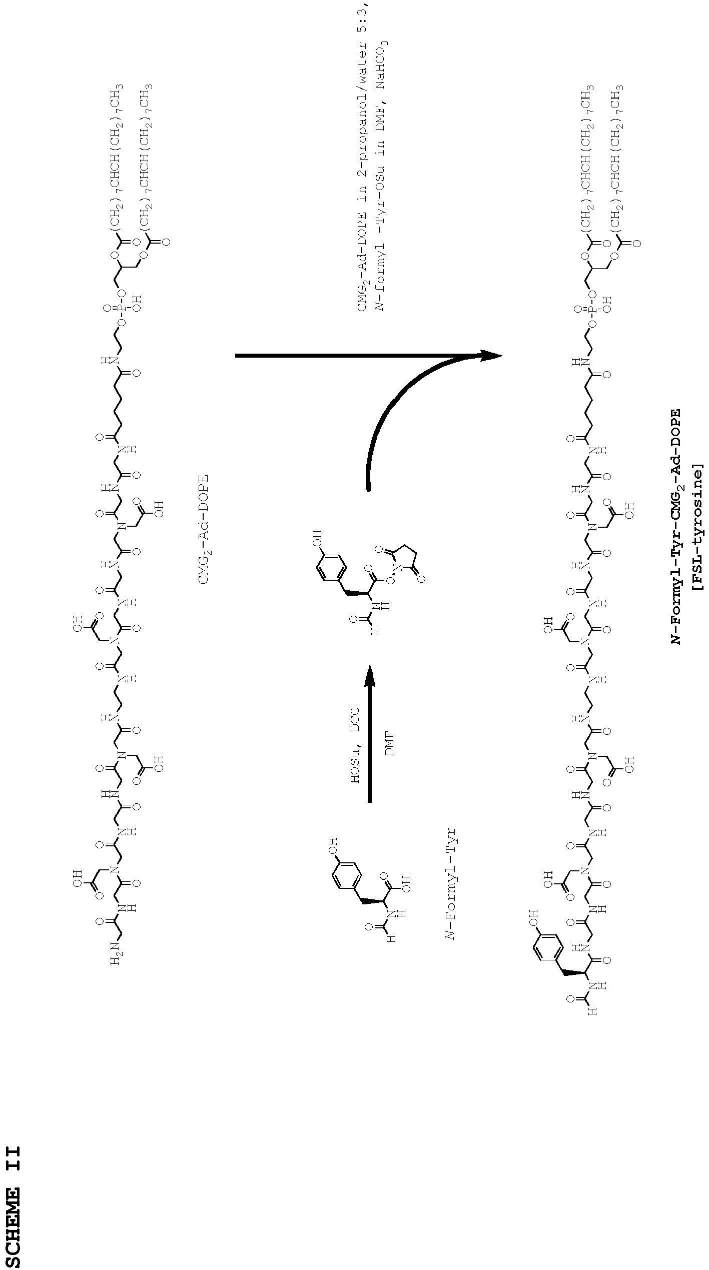

### Preparation of the construct designated FSL-fluorescein

The construct was prepared according to Scheme I as previously described (Korchagina *et al* (2008)) (Route 1) or as follows (Route 2) :

### Preparation of activated 1,2-O-distereoyl-sn-glycero-3-phosphatidylethanolamine (DSPE) and activated 1,2-O-dioleoyl-sn-glycero-3-phosphatidylethanolamine (DOPE) (L-A)

To a solution of bis(N-hydroxysuccinimidyl) adipate (A) (70 mg, 205 µmol) in dry N,N-dimethylformamide (1.5 ml) were added DOPE or DSPE (L) (40 µmol) in chloroform (1.5 ml) followed by triethylamine (7 µl). The mixture was kept for 2 h at room temperature, then neutralized with acetic acid and partially concentrated *in vacuo.*

Column chromatography (Sephadex LH-20, 1:1 chloroform-methanol, 0.2% acetic acid) of the residue yielded the activated lipid (L-A) (37 mg, 95%) as a colorless syrup; TLC (chloroform-methanol-water, 6:3:0.5) : R_{f} = 0.5 (DOPE-A), R_{f} = 0.55 (DSPE-A).

¹H NMR (CDCl_{3/}CD₃OD, 2:1), δ:
DSPE-A - 5.39 (m, 1H, -OCH₂-C*H*O-CH₂O-), 4.53 (dd, 1H, J=3.42, J=11.98, -CCOOHC*H*-CHO-CH₂-), 4.33 (dd, 1H, J=6.87, J=11.98, - CCOO*H*CH-CHO-CH₂O-), 4.23 (m, 2H, PO-C*H*₂-CH₂-NH₂), 4.15 (m, 2H, *-CH₂*-OP), 3,61 (m, 2H, PO-CH₂-C*H₂*-NH₂), 3.00 (s, 4H, ONSuc), 2.81 (m, 2H, -C*H₂*-CO (Ad), 2.48 (m, 4H, 2x (-C*H₂*-CO), 2.42 (m, 2H, -C*H₂*-CO (Ad), 1.93 (m, 4H, COCH₂C*H*₂C*H*₂CH₂CO), 1.78 (m, 4H, 2x (COCH₂C*H*₂-), 1,43, 1.47 (2 bs, 40H, 20CH₂), 1.04 (m, 6H, 2CH₃).

DOPE-A - 5.5 (m, 4H, 2x(-C*H=*C*H-*)*,* 5.39 (m, 1H, -OCH₂-C*H*O-CH₂O-), 4.58 (dd, 1H, J=3.67, J=11.98, -CCOOHC*H*-CHO-CH2O-), 4.34 (dd, 1H, J=6.61, J=11.98, -CCOO*H*CH-CHO-C*H₂*O-), 4.26 (m, 2H, PO-C*H*₂-CH₂-NH₂), 4.18 (m, 2H, -C*H*₂-OP), 3.62 (m, 2H, PO-CH₂-C*H₂*-NH₂), 3.00 (s, 4H, ONSuc), 2.8 (m, 2H, -C*H₂*-CO (Ad), 2.50 (m, 4H, 2x(-C*H₂*-CO), 2.42 (m, 2H, -C*H₂*-CO (Ad), 2.17 (m, 8H, 2x(-C*H₂*-CH=CH-C*H₂*-), 1.93 (m, 4H, COCH₂C*H₂*C*H₂*CH₂CO), 1.78 (m, 4H, 2x(COCH₂C*H₂*-), 1,43, 1.47 (2 bs, 40H, 2OCH₂), 1.04 (m, 6H, 2CH₃).

### Condensation of activated DOPE (L-A) with cadaverine

To a solution of cadaverine (110 µl, 940 µmol) in dichloromethane (3 mL) a solution of activated DOPE (L-A) (90 mg, 93 µmol) in dichloromethane (CH₂Cl₂) (0.5 mL) was added. The mixture was kept for 0.5 h at room temperature and then portions of 180 µL of acetic acid were added over the course of 5 minutes. The mixture was concentrated *in vacuo* and the residue chromatographed (Sephadex LH-20, 1:2 chloroform-methanol) to provide 74 mg (84 %) of DOPE-Ad-cadaverine, R*_{f}* 0.35 (ethyl acetate-isopropanol-water, 2:3:1)

¹H NMR (700 MHz, [D₃]CHCl₃/[D₄]CH₃OH 1:1, 30°C): δ, ppm 5.50 (m, 4H, 2x(-CH=CH-), 5.38 (m, 1H, -OCH₂-CHO-CH₂O-), 4.59 (dd, 1H, J=6.6, *J_{gem}*=11_{.}8*,* HHC-O-C(O)-), 4.35 (dd, 1H, *J*=3.2, J*_{gem}*=11*.*8*,* HHC-O-C(O)-), 4.14 (m, 2H, -OCH-CH₂-O-P-), 4.08 (m, 2H, -P-O-CH₂-CH₂-NH-), 3.58 and 3.39 (2m, 2x2H, N- CH₂-CH₂-O-P- and N-CH₂-(CH₂)₃-CH₂NH₂) , 3.05 (m, 2H, N-CH₂-(CH₂)₃-CH₂NH₂), 2.48 (m, 4H, 2x(-CH₂-CO), 2.39 (m, 4H, COCH₂CH₂CH₂CH₂CO), 2.19 (m, 8H, 2x(-CH₂-CH=CH-CH₂-), 1.8, 1.72, 1.58 (3m, 10H, 2H, 2H, COCH₂CH₂CH₂CH₂CO, 2x(COCH₂CH₂-), and-N-CH₂-(CH₂)₃-CH₂NH₂), 1.42, 1.46 (2 bs, 40H, 20 CH₂), 1.05 (m ∼t, *J*≈7 Hz 6H, 2 CH₃) .

### Condensation of DOPE-Ad-cadaverine with fluorescein isothiocyanate (FITC)

To a solution of fluorescein isothiocyanate (FITC) (16.3 mg, 42 µmol) in DMSO (0.3 mL) a solution of DOPE-Ad-cadaverine (40.2 mg, 42 µmol) in dichloromethane (0.5 mL) and triethylamine (7 µL) were added. The mixture was kept for 2 h at room temperature and then partially concentrated *in vacuo.* The residue was chromatographed (Sephadex LH-20, 1:2 chloroform-methanol) to provide 8 mg (∼8%) of FSL-fluorescein (triethylamine(Et₃N) salt) and about 40 mg of crude FSL-fluorescein. The latter was chromatographed (silica gel, ethyl acetate-isopropanol-water, 6:3:1) to yield 24.3 mg (∼ 40%) of FSL-fluorescein (sodium (Na) salt). The NMR spectra of the obtained salts of FSL-fluorescein were identical to those of the corresponding salts obtained via Route I.

### Preparation of the construct designated FSL-tyrosine (FSL-Tyr)

The construct was prepared according to Scheme II as follows:

### Preparation of N-oxysuccinimide ester of N-formyl-tyrosine

N-formyl-tyrosine (81.2 mg) was dissolved in DMF (1.6 mL) followed by the addition of N-hydroxysuccinimide (60 mg) and dicyclohexylcarbodiimide (80 mg in 0.8 mL DMF). The mixture was stirred for 2.5 h at room temperature and the precipitate of *N-*dicyclohexylurea filtered off. *N*-formyl-tyrosine (R*_{f}* 0.53), *N-*formyl-tyrosine *N*-oxysuccinimide ester (R*_{f}* 0.64) (CHCl₃/MeOH/AcOH; 40:20:3 (v/v/v)).

### Preparation of N-formyl-tyrosine-CMG₂-Ad-DOPE

The intermediate CMG₂-Ad-DOPE was prepared as previously described (Bovin *et al* (2009)). CMG₂-Ad-DOPE (250 mg) was dissolved in 5 mL of water and 3 mL of methanol. A solution of the *N*-oxysuccinimide ester of *N*-formyl-tyrosine, 1 mL of DMF and 0.647 mL of 1 M NaHCO₃ were then added. The mixture was stirred for 45 min, than acidified with 0.2 mL of AcOH. The solution was evaporated *in vacuo* to a minimal volume. Following the addition of 200 µL of Py, the residue was separated on an LH-20 column (170 ml) (2-propanol/water; 1:2 + 0.05 M Py·HOAc. Fractions containing pure product were combined, evaporated and freeze-dried.

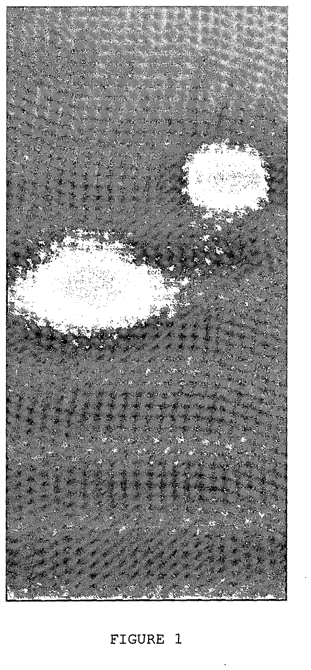

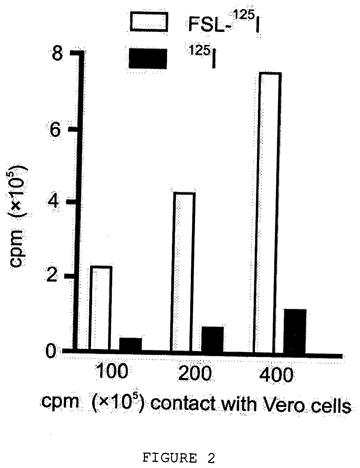

The residue (Na₁, Py₁ salt) was dissolved in 3 mL of water. A 60 µL volume of 8 mM NH₃ was then added and the resulting solution freeze-dried. Yield 239 mg (91%). R*_{f}* = 0.53 (CHCl₃/MeOH/water; 3:6:1 (v/v/v)). Brutto formula C91H156N20NaO33P. molecular weight as Na₁(NH₄)₄ salt - 2112.3. MALDI TOF mass-spectrum (FLEX-PC, DHB) *m*/*z*: 2044 M+Na; 2060 M+K; 2066 M+2Na; 2082 M+Na+K; 2088 M+3Na; 2098 M+2K (Figure 1). ¹H-NMR spectrum, δ ppm (5 mg/ml in CD₃OD/D₂O 1:2, 303K, 800MHz): 8.238 (s, 1H, H(CO)N), 7.324 and 6.993 (d, 2x 2H, J = 8.2 Hz, *p*-C₆H₄), 5.507 (m, 4H, 2 CH=CH), 5.453 (m, 1H, OCH₂-CH-CH₂O), 4.823 (∼t, 1H, J = 7.2 Hz, αCH of tyrosine), 4.615 (dd, 1H, J₁ = 12 Hz, J₂ = 2 Hz, OCH₂-CH-CHO), 4.442-4.063 (37H, 32H of CMG₂, OCH₂-CH-CHO and OCH₂CH₂N), 3.523-3.478 (4H, NCH₂CH₂N), 3.251 (dd, 1H, J_{β} = 13.8 Hz, J_{α} = 6.3 Hz, βCH of tyrosine), 3.138 (dd, 1H, J_{β} = 13.8 Hz, J_{α} = 8.1 Hz, βCH' of tyrosine), 2.532-2.453 (m, 8H, 4 C(O)CH₂), 2.189 (m, 8H, 2 CH₂CH=CHCH₂), 1.802 (m, 4H, 2 C (O)CH₂CH₂ of adipic fr.), 1,771 (m, 4H, 2 C(O)CH₂CH₂ of DOPE), 1.514, 1.477 and 1.459 (m, 40H, 20 CH₂ of DOPE), 1.064 (t, 6H, J = 7 Hz, 2 CH₃) (Figure 2).

### Preparation of the constructs designated FSL-Tyr4 and FSL-Tyr8

The constructs were prepared according to Scheme III as follows:

### Solid-phase synthesis (SPS) of multivalent tyrosine dendrons

Solid-phase reactions were performed manually in 50, 30 or 20 mL polypropylene (PP) syringes (BD, USA) equipped with PP frit at the bottom and 19G disposable needle used for connecting Luer outlet to the waste flask via the septum stopper during washing steps. The needle was changed for Luer stopper in the course of coupling and deprotection steps performed with occasional shaking. Volumes of solvents and reagent solutions used for every SPS operation were *circa* 10 mL /1 g of starting resin initially, the volume gradually increased to accommodate swelling of the peptide resin (PR) due to incorporation of peptide material. By default, during the course of all repeated coupling steps DMAP was introduced 30 to 60 min before the reactions were terminated (10 mol% with respect to resin loading). Completeness of the coupling reactions was monitored by the qualitative ninhydrin reaction (Kaiser *et al* (1970)). Resin loadings (mmol/g) were calculated on the basis of respective PR weight gain.

### Preparation of Boc-NHCH₂CH₂SCH₂-resin (Resin I) and (Lys)₂-Lys-NHCH₂CH₂SCH₂-resin (PR II) (Scheme III - Part 1)

A vigorously stirred slurry of finely grounded cysteamine (1.05 g, 13.6 mmol) in DMF (10 ml) was treated with Boc₂O (3.56 g, 16.4 mmol) until starting material completely dissolved (*circa* 0.5 hrs). (The dissolution may require brief heating at 40 to 50 °C.) To the solution of N-Boc-cysteamine obtained diisopropylethylamine (DIEA; 1.5 mL, 7 mmol), NaI (102 mg, 0.7 mmol) and DMF were added to final volume of 60 mL, followed by 10 g (6.8 mmol) of chloromethylated copolymer of styrene; 1% divinylbenzene (0.68 meq of Cl/g; Peptide Institute Inc., Japan). The alkylation reaction was allowed to proceed for 2 hours at room temperature and then at 50 °C overnight. The resin was washed with IPA-DMF (2:1), DMF, IPA-DMF (2:1), DMF, IPA-DMF (2:1), dichloromethane (DCM), IPA-DCM (1:1, v/v), DCM and dried in vacuum to provide 10.96 g Boc-NHCH₂CH₂SCH₂-resin (Resin I) with loading of 0.61 mmol/g estimated from the observed weight gain and 0.03 mmol/g of residual chloromethyl functions quantitated according to the published procedure (Tam (1985)). A 2 g (1.22 mmol) amount of the Resin I was treated with TFA-DCM (1:1, v/v) for 1 min and 25 min, DCM, DMF-IPA (1:2), 2 times [DIEA(5% v/v) in DCM, DMF-IPA (1:2)] followed by solution of Boc-Lys(Boc)-OBt. The latter was prepared by dissolving 0.63 g, (1.2 mmol) of Boc-Lys(Boc)-OH*DCHA in the solution of TBTU (0.35 g, 1.1 mmol) in DMF (5 mL) with sonication. To an essentially clean solution 0.16 g, (1.2 mmol) of HOBt and DMF were added to provide a final volume of 15 mL. Coupling was terminated after 2 hours and the resin was washed with DIEA (5% v/v) in DCM, DMF-IPA (1:2) and the residual amino groups were acetylated with Ac₂O-DMF (1:4 v/v) until negative ninhydrin test obtained. The resin was treated twice with TFA-DCM (1:1, v/v) for 1 min and 40 min, DCM, DMF-IPA (1:2), 2 times [5 % (v/v) DIEA in DCM, DMF-IPA (1:2)] and coupling carried out for 5 hours with Boc-Lys(Boc)-OBt solution obtained as described above from respective DCHA-salt (1.27 g, 2.4 mmol), TBTU (0.69 g, 2.16 mmol) and HOBt (0.16 g, 1.2 mmol). The resin was washed with 5% (v/v) DIEA in DCM, DMF-IPA (1:2) and coupling was repeated with the fresh solution of Boc-Lys(Boc)-OBt at 50 °C overnight. The resin washed with DMF-IPA (1:2), DCM, DCM-IPA (1:2, v/v)] was treated with TFA-DCM (1:1, v/v) for 1 min and 60 min, DCM, DMF-IPA (1:2), 2 times [5% (v/v) DIEA in DCM, DCM-IPA (1:2)] and dried in vacuum to constant weight; yield 2.81 g of PR II with loading 1.7 mmol NH₂/g.

### Preparation of [Suc-Tyr(Bzl)]₈- (Lys) ₄- (Lys) ₂-Lys-NHCH₂CH₂SCH₂-Resin (PR III) and (Suc-Tyr)₈-(Lys)₄-(Lys)₂-Lys-NHCH₂CH₂SH (Tyr₈-dendron) (Scheme III - Part 2A)

1 g (1.7 mmol) of dry PR II was treated with oxybenzotriazol ester solution prepared as described above from Boc-Lys(Boc)-OH*DCHA-salt (2.1 g, 3.9 mmol), TBTU (1.12 g, 3.5 mmol) and HOBt (0.24 g, 1.7 mmol) for 6 hours at 50 °C. A second coupling was performed with double quantity (7.8 mmol) of the active ester at 50 °C overnight. Ninhydrin negative resin was washed with DCM, DCM-IPA (1:2), deprotected (1 min and 70 min) and neutralized as above. The next coupling was repeated 3 times with a solution of 6.4 g (13 mmol) of Boc-Tyr(Bzl)-ONp (Reanal, Hungary) in 10 mL DMF containing 0.45 g (3.3 mmol) HOBt. The first coupling was performed at room temperature for 5 hours, the second and the third couplings were performed at 50 °C for 6 and 12 hours, respectively. The ninhydrin-negative resin obtained was washed with 2 times [DMF-IPA (1:2), 5% (v/v) DIEA in DCM], DCM, DCM-IPA (1:2), deprotected (1 min and 60 min) and neutralized as above. The final acylation with 1 g (10 mmol) of succinic anhydride (Pierce) in DMF (10 mL) was carried out for 5 hours at room temperature and repeated with fresh reagent at 50 °C for 6 and 12 hours. The PR III was thoroughly washed with DMF-IPA (1:2), DCM, DMF, DCM-IPA (1:2), DCM to remove traces of p-nitrophenol and dried in vacuo; yield 1.52 g. A part of the PR III (1.05 g) was subjected to 'low-high' HF cleavage procedure (Tam and Merrifield, (1987)). "Low-HF" procedure details: 12 mL DMS/5 mL HF, 0 °C, 2 hrs. Volatiles were removed in vacuum and the solids were washed repeatedly with DCM and EtOAc to ensure that any traces of benzyldimethylsulfonium by-product are removed completely, and dried. "High-HF" cleavage was performed in 10 mL of HF-p-cresol (9:1, v/v) for 2 h at 0 °C. After HF removal the residue was triturated with Et₂0 and washed 3 times [EtOAc, Et₂O] (both solvents contained 1 % (v/v) of mercaptoethanol) and peptide material was extracted with 3 times 6 mL of DCM-TFE (2:1). The extract was concentrated in vacuum, peptide precipitated with Et₂0, centrifuged and re-precipitated two times with Et₂0 from a minimum volume of DCM-TFE(2:1). Yield - 170 mg of Tyr₈-dendron which was characterized by ESI-MS and NMR and used in the next step without further purification. ESI-MS (Negative mode, 20% MeOH),m/z: 1539,3 [M-2H]²⁻; 1025,9 [M-3H]³⁻. Calculated MW 3080.4

### Preparation of (Suc-Tyr)₈-(Lys)₄-(Lys)₂-Lys-NHCH₂CH₂S-Mal-βAla-CMG₂-Ad-DOPE (FSL-Tyr8) (Scheme III - Part 3A)

A solution of a (Suc-Tyr)₈-(Lys)₄-(Lys)₂-Lys-NHCH₂CH₂SH (20.7 mg, 6.2 µmol) in 6 mL 0.1M NMM formate in 30% aqueous isopropyl alcohol (30% IPA) pH 6.6 was combined with 1.2 mL of the same buffer, in which 9.02 mg, 4.5 µmol of Mal-βAla-CMG₂-Ad-DOPE (Bovin *et al* (2009)) were dissolved. The clear solution was kept overnight at room temperature and lyophilized. The residue thus obtained was triturated with IPA (1 mL) and the mixture was sonicated until uniform suspension was obtained (*circa* 10 min) . The slurry was then transferred into an Eppendorf tube and centrifuged. The precipitate was further washed with IPA, redissolved in 200 µL 20% IPA, re-precipitated with 1 mL of IPA and washed alternately with EtOH and IPA (2 times 200 µL of each, ultrasonic bath followed by centrifugation). The wet precipitate was dissolved in 2 ml of water and lyophilized; yield 20.2 mg (88%) of amorphous white powder. ESI-MS (Negative mode, 20% IPA), m/z: 1686.6 [M-3H]³⁻, 1264.7 [M-4H]⁴⁻; 1271.4 [M-4H+Na]⁴⁻; Calculated MW 5062.6.

### Preparation of (Suc-Tyr)₄-(Lys)₂-Lys-NHCH₂CH₂SCH₂-resin (PR IV) and (Suc-Tyr)₄-(Lys) ₂-Lys-NHCH₂CH₂SH (Tyr₄-dendron) (Scheme III - Part 2B)

The title PR IV was assembled on PR II (1 g, 1.7 mmol) via two chain extension steps detailed in the preparation of PR III applying the same molar excesses of activated species with respect to resin. Yield of PR IV - 1.39 g. A part of the PR IV (1.11 g) obtained was subjected to 'low-high' HF cleavage procedure as specified above for octavalent homologue to yield 117 mg of Tyr₄-dendron. ESI-MS (Negative mode, 30%IPA),m/z: 1513.8 [M-H]⁻; 757.5 [M-2H]²⁻ Calculated MW 1514.7

### Preparation of (Suc-Tyr)₄-(Lys)₂-Lys-NHCH₂CH₂S-Mal-βAla-CMG₂-Ad-DOPE (FSL-Tyr4) (Scheme III - Part 3B)

The construct FSL-Tyr4 was obtained according to standard conjugation procedure exemplified for the construct FSL-Tyr8 as described above. Reaction of 15.1 mg (10 µmol) of (Suc-Tyr)₄-(Lys)₂-Lys-NHCH₂CH₂SH with 13.23 mg (6.6 µmol) of Mal-βAla-CMG₂-Ad-DOPE in 5.5 mL of 30%v/v IPA containing 0.1M NMM formate, pH 6.6 yielded 17.5 mg (76%) of the desired construct FSL-Tyr4. ESI-MS (Negative mode, 30%IPA), m/z: 1746,7 [M-2H]²⁻; 1164,7 [M-3H]³⁻, 1172,2 [M-3H+Na]³⁻; 873,3 [M-4H]⁴⁻; 698,6 [M-5H]⁵⁻; Calculated MW 3496.9.

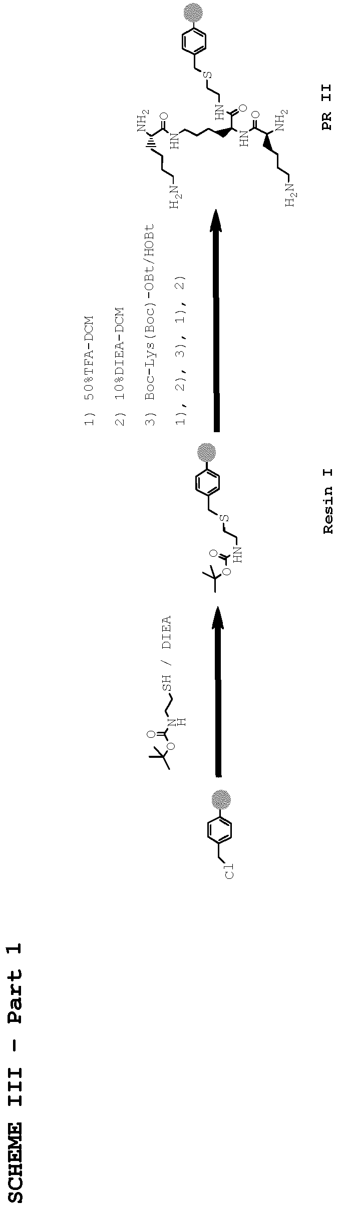

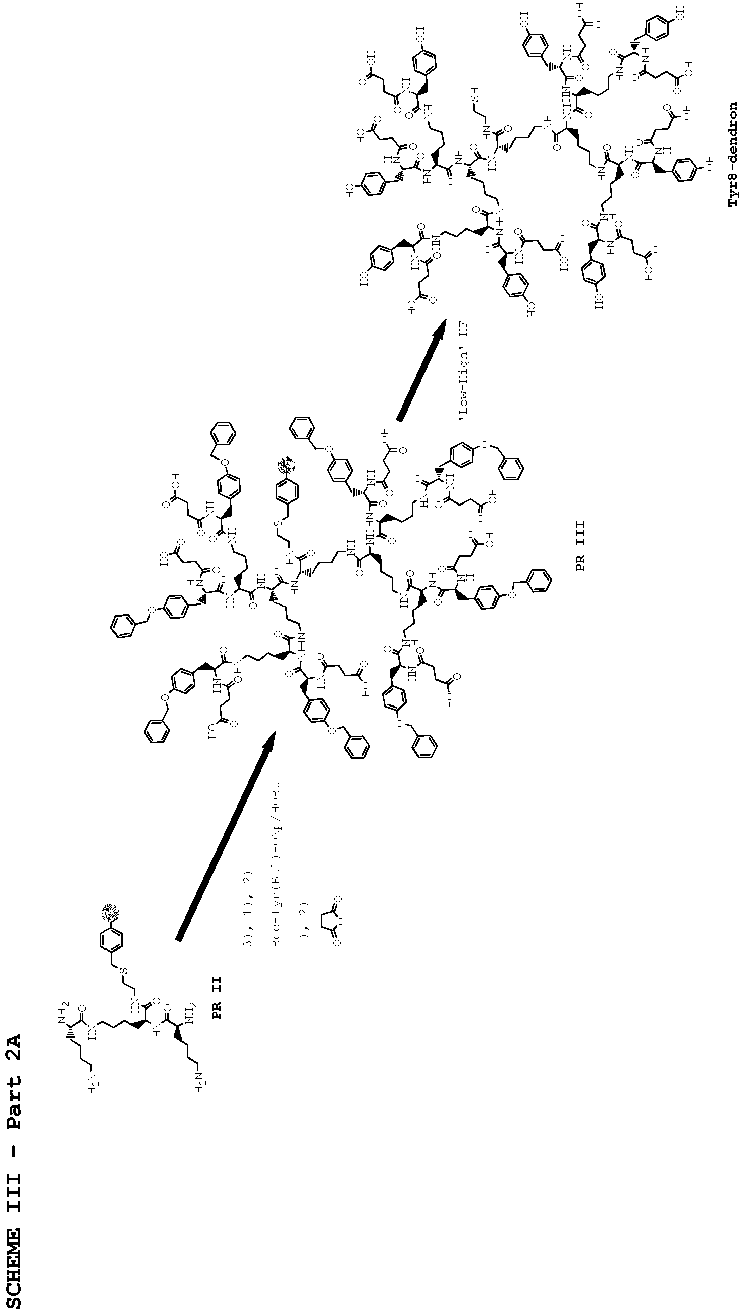

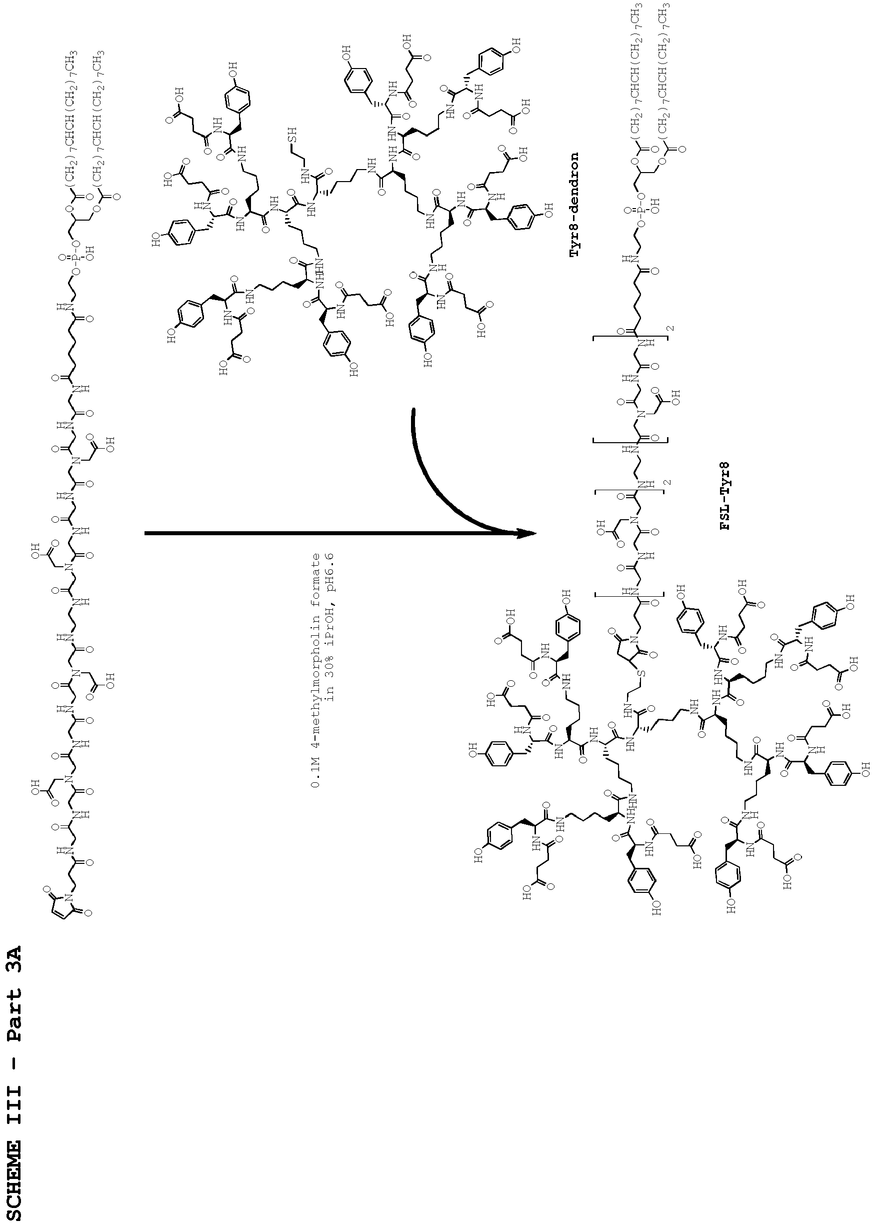

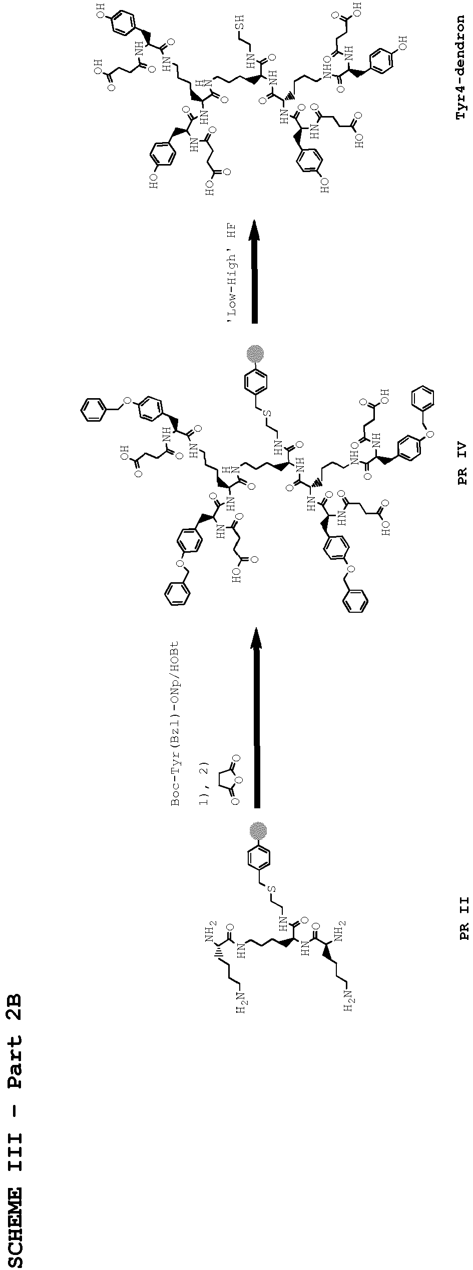

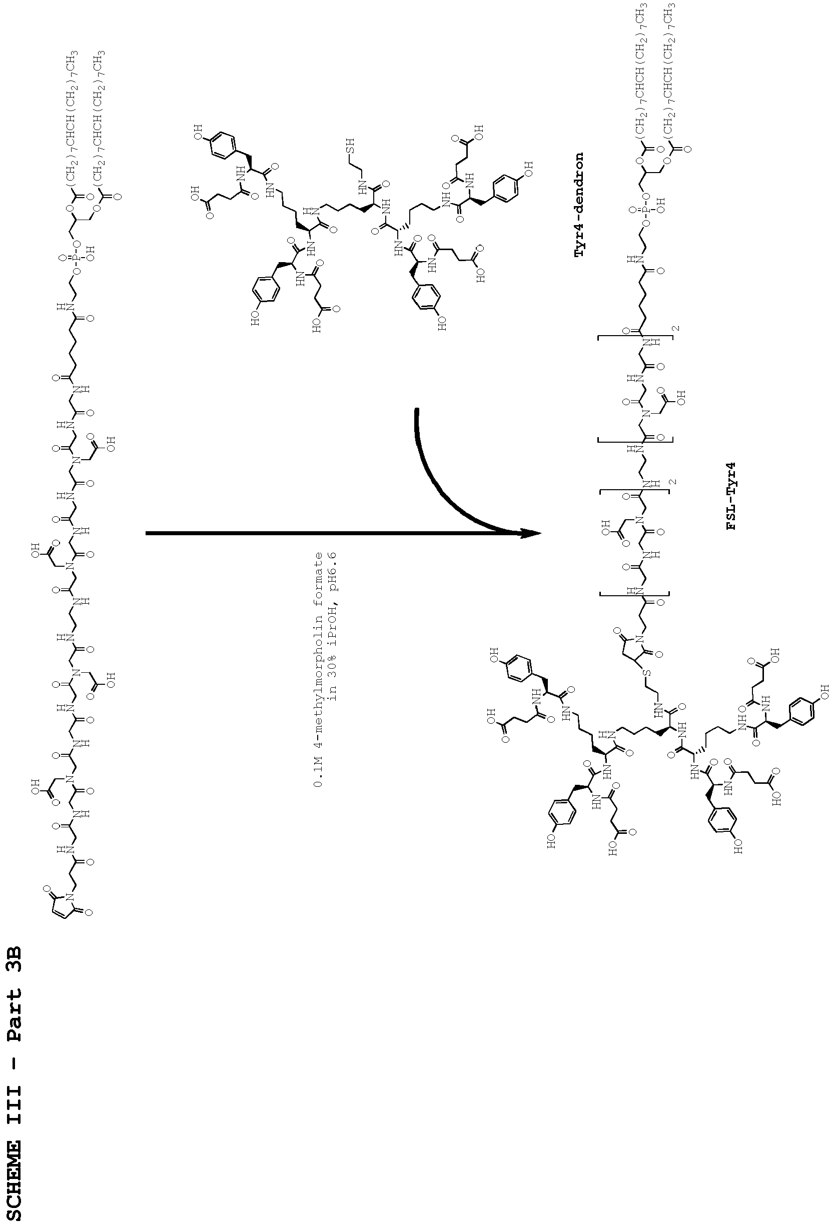

The following procedures and results are also described and discussed in the publication of Hadac *et al* (2011).

### Iodination of the construct FSL-Tyr

To oxidatively label the tyrosine residue of the construct we Pierce (Cat. 28601, Thermo Fisher Scientific Inc., Rockford, IL) iodination tubes were used. Briefly, a Pierce Iodination tube was wetted with 1 ml Tris-HCl-NaCl buffer (25 mM Tris-HCl pH 7.5, 0.4 M NaCl) and decantanted. In a well vented fume hood, 100 µl Tris-HCl-NaCl buffer followed by 1 mCi Na¹²⁵I (Cat. NEZ-033A PerkinElmer, Cambridge, MA) was added and incubated at room temperature for 6 minutes swirling every 30 seconds. The activated Na¹²⁵I was transferred to a new tube containing the construct designated FSL-tyrosine (0.3 mg/150 µL) and incubated at room temperature for 8 minutes, swirling every 30 seconds. The solution was then transferred into the sample reservoir of a Microcon-10 filter device (Millipore Corporation, Billerica, MA) and 250 µL Tris-HCl-NaCl buffer added to the reservoir, sealed and spun in a microcentrifuge in a fume hood for 30 minutes at 13,000 rpm. The flow through (FT1) was collected and 300 µl Tris-HCl-NaCl buffer added to the sample reservoir. The centrifugation was repeated with collection of the flow through (FT2) and retained sample (iodinated(¹²⁵I) FSL-tyrosine). All fractions were quantified in a dose calibrator and aliquots of the iodinated (¹²⁵I) FSL-tyrosine stored at -20 °C.

### Modification of VSV with the construct designated FSL-fluorescein

A volume of 1.25 µL of FSL-fluorescein (20 or 200 µg/ml) to provide a final concentration of 1 and 10 µg/ml was added to 25 µL of VSV (7.8 x 10¹⁰ TCID₅₀ units/mL). The suspension of VSV and FSL-fluorescein were incubated for 2 hours at 37 °C. Sample of VSV modified to incorporate the construct (VSV-FSL-FLR04) and control samples of virus were then fixed with 4% paraformaldehyde on ice for 10 minutes.

### Modification of H1Ñ1 with the construct designated FSL-fluorescein

0.5 mg of FSL-fluorescein was dispersed in 50 µL 50 mM NaHCO₃, pH 8.3 followed by addition of 450 µL 0.1M PBS. The obtained stock solution was used for preparation of three concentrations; 25, 100, and 200 µg/mL. To modify virus 60 µL of the stock solution was added to an equal volume of virus preparation and incubated at room temperature for 1 h. An excess of FSL-fluorescein was removed using chromatography on Sephadex G-50. Modified virus was stored in 0.1 M PBS containing 0.01% NaN₃.

### Influenza virus (H1N1) infectivity test

Infectivity of H1N1 was tested on swine testicles cell culture (ST cells). A 24-well plate with cells was washed with 1% BSA in PBS (PBA) followed by infection with 200 µl virus preparation (200 HAU) in PBS. Cells were incubated with virus at 37 °C for 1 h followed by washing two times with PBA. The cell monolayer was treated with trypsin solution and the obtained cell suspension washed with PBA and fixed in 0.1% formaldehyde solution in PBS. Cell fluorescence was measured using a laser flow cytometer DACO Galaxy (Denmark) at 488 nm.

### Modification of MV-NIS by FSL-fluorescein and subsequent labelling of KAS6/1 cells

A volume of 5 µL of FSL-fluorescein (100 µg/ml) was added to 100 µL of MV-NIS (1.5 x 10⁸ TCID₅₀ units/mL) and incubated for 2 hours at 37 °C to prepare MV-FSL-FLR04. The suspension was then diluted to 250 µl with PBS. KAS6/1 cells were grown in RPMI 1640 supplemented with heat-inactivated 10% fetal bovine serum, 100 U/mL penicillin, 100 mg/mL streptomycin and interleukin-6 (IL-6, 1 ng/mL). A volume of 140 µL of the MV-FSL-FLR04 preparation was added to 100 µL of a KAS6/1 cell suspension (4 x 10⁶/100 µL) in OptiMEM (Invitrogen, Carlsbad CA) media and incubated at 37 °C for 1 hour. Following incubation the resultant MV-FSL-FLR04 labelled KAS6/1 cells were pelleted and washed once (PBS with 1% BSA) by centrifugation and fixed on ice for 10 minutes in 4% paraformadehyde.

### Modification of Vero cells with iodinated (¹²⁵I) FSL-Tyr

In 6-well tissue culture plates 5 x 10⁵ Vero cells maintained in Dulbecco-modified Eagle medium containing 10% fetal bovine serum (American Type Culture Collection, Manassas, VA) per well were grown to confluence overnight then washed with HBSS 10 mM HEPES pH 7.3 with KOH. A volume of 1 mL of HBSS-HEPES (10 mM HEPES, pH 7.3) was added to a monolayer followed by varying amounts of the iodinated construct or ¹²⁵I to give 1, 2 or 4 x 10⁷ cpm per well. After a 2-hour incubation at 37 °C the plate was placed on ice, the supernatant aspirated, and the monolayer washed with 1 ml of iced HBSS buffer. Cells were lysed with 1 ml of 1M NaOH and each lysate transferred to a test tube for quantitation of radioactivity.

### Monitoring distribution in vivo

To determine the distribution of labelled virus in mice 200 µl of VSV-hIFNβ (5.8 x 10¹⁰) was added to 150 µCi of iodinated (¹²⁵I) FSL-tyrosine and incubated for 4 hours at room temperature. Syringes were then loaded each with a total of 50 µCi of labelled virus (VSV-FSL-¹²⁵I). Control syringes were also loaded each with 50 µCi, two with Na¹²⁵I and two with iodinated (¹²⁵I) FSL-tyrosine (FSL-¹²⁵I). Each of seven mice were injected IP with one of the ¹²⁵I reagents. Prior to imaging mice were anesthetized with inhalation of isoflurane and imaged 1 hour post sample injection using micro-SPECT/CT system (X-SPECT, Gamma Medica Ideas) and image analysis using PMOD software (PMOD Biomedical Image Quantification and Kinetic Modeling Software, PMOD Technologies Zurich, Switzerland). One mouse from each sample group underwent additional imaging at 4 and 24 hour time-points. After each final imaging point the mouse was sacrificed, dissected and radioactivity in organs was quantified in a dose calibrator.

### FACScan flow cytometry

Samples were analyzed by FACScan flow cytometry and Cell Quest Pro software (BD Biosciences, San Jose, CA).

### Quantitation of radioactivity

Radioactivity was quantitated with an Isodata-10 gamma spectrometer (Polymedio, Yorktown Heights, NY) and radioisotope calibrator (model CRC-5R, Capintec, Inc. Ramsey, NJ).

### RESULTS

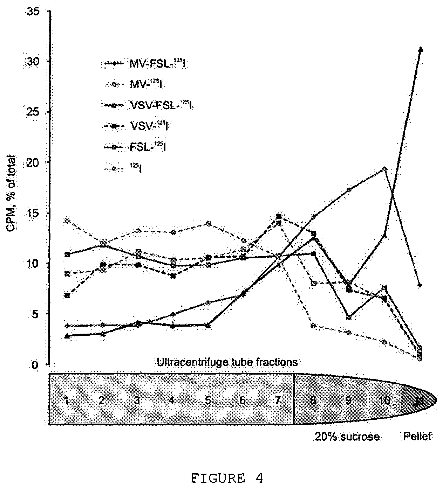

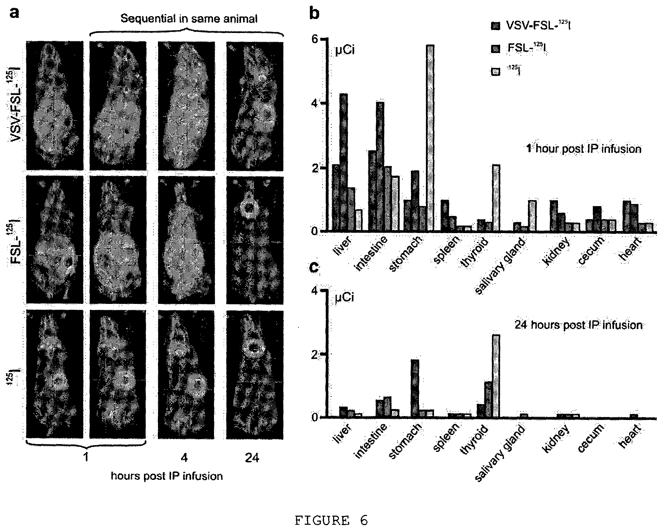

The iodinated (¹²⁵I) form of the construct designated FSL-tyrosine (FSL-¹²⁵I) had the spontaneous and controlled membrane insertion character of other FSL constructs. Its ability to label Vero cells has been investigated and contrasted with direct ¹²⁵I labelling. It has been shown that at equivalent radioactive counts, FSL-¹²⁵I was about 10-fold more effective at labelling cells than ¹²⁵I and the labelling occurred in a concentration dependent manner (Figure 4). A comparison of ¹²⁵I and FSL-¹²⁵I labelling of MV and VSV, and subsequent sucrose gradient purification (Figure 5) showed that more FSL-¹²⁵I labelled virions than ¹²⁵I labelled virions, could be isolated in the sucrose fraction, compared with the buffer, where the unbound label is predominantly found (Figure 6).

VSV was determined to be labelled with FSL-FLR04 in a manner similar to cells (Blake *et al* (2010)) by contacting VSV with the FSL-FLR04 to create modified virus (VSV-FSL-FLR04) and analysing by FACScan (Figure 7(a)). By simply increasing the concentration of FSL construct, the intensity of modified virions increased (Figure 7(a)). Similarly, H1N1 modified with FSL-FLR04 showed a concentration dependent modification (Figure 7(b)). For comparison H1N1 was labelled by the conventional direct FITC method (Yoshimura and Ohnishi (1984)) which gave about 9-times greater total fluorescence than H1N1 modified with 25 µg/ml FSL-FLR04 (Figure 7(b)). However, when these labelled and modified H1N1 virions were allowed to infect ST cells, essentially the same level of fluorescence was seen for 25 µg/ml FSL-FLR04 modified as with direct FITC-labeled H1N1 (Figure 7(c)). This result, starting with the 9-times more fluorescence of direct FITC labelled H1N1 yet obtaining only equivalent fluorescence with the 25 µg/ml FSL-FLR04 moified H1N1 post infection of MDCK cells, can be interpreted to mean that 9-times less FITC labelled virions infected the MDCK cells. Thus, the FSL-FLR04 labelled virions appears to conserve infective potency better than FITC-labelled virions, perhaps, due to the non-covalent nature of the modification. When a higher concentration FSL-FLR04 construct modified H1N1 was used to infect MDCK cells (e.g. 100 and 200 µg/ml) increased fluorescent intensity was obtained (Figure 7(b)). MV was modified with FSL-FLR04 (MV-FSL-FLR04) and then allowed to react with a KAS6/1 cell line suspension (Figure 7(d)). These results, like those for H1N1 (Figure 7(c)) show that the modified viruses retain their ability to bind to cells, and become detectable by flow cytometry. The additional small peak seen with KAS6/1-MV-FSL-FLRO4 cells (Figure 7(d)) is probably due to dead cells, as unbound virus was excluded by gating.

Following peritoneal infusion we compared the distribution *in vivo* of ¹²⁵I and FSL-¹²⁵I construct with that of VSV-FSL-¹²⁵I (Figure 8). Radioactive iodine has a very well described distribution *in vivo* (Hammond *et al* (2007)) to thyroid, salivary glands, stomach and bladder and the ¹²⁵I results were concordant with the literature. Within 1 hour post administration to the peritoneal cavity labelled virions (VSV- FSL-125I), in contrast to ¹²⁵I and FSL-¹²⁵I, localized to liver, spleen and bloodstream - with some signal also retained in the peritoneal cavity (Figure 8(a) and 8(b)). Intravenously administered VSV-FSL-¹²⁵I localized similarly, but without the strong peritoneal signal (not shown). FSL-¹²⁵I alone followed a similar distribution to labelled virus, but was significantly different to free ¹²⁵I, which rapidly distributed to the stomach and then to the thyroid (Figure 8(a)). Due to the variable nature of urine collection bladder quantitation was not considered. At 4-hours post infusion the distribution (Figure 8(a)) had not changed significantly from the 1 hour time point although more systemic spread had occurred. At 24 hours (Figure 8(a) and 8(c)) both ¹²⁵I and FSL-¹²⁵I injected animals gave essentially only thyroid distribution while the VSV-FSL-¹²⁵I injected animals had a distribution equivalent to the 1 hour distribution of ¹²⁵I. The accumulation of radioactive iodine in the thyroid from both VSV-FSL-¹²⁵I and FSL-¹²⁵I is not surprising and suggests that degradation of the FSL-¹²⁵I had occurred releasing free iodide, which then was concentrated in the thyroid gland.

The substitution of the less favoured particle emitting radioisotope ¹³¹I for ¹²⁵I in any of the disclosed methods is part of the disclosure.

### REFERENCES

Barr et al (2010) Function-Spacer-Lipid (FSL) constructs enable inkjet printing of blood group antigens FEBS J, Vol 277 (S1), 235.
Blake *et al* (2003) *Embryo modification and implantation* international application no. PCT/NZ03/00059 (publ. no. WO 03/087346).
Blake et al (2010) Fluorophore-kodecytes - fluorescent function-spacer-lipid (FSL) modified cells for in vitro and in vivo analyses FEBS J, Vol 277 (S1), 199.
Bovin *et al* (2009) *Functional lipid constructs* international application no. PCT/NZ2008/000266 (publ. no. WO 2009/048343).
Campos et al (2004) Avidin-based targeting and purification of a protein IX-modified, metabolically biotinylated adenoviral vector Mol Ther, Vol 9, 942-954.
Chesla et al (2010) Solid phase syphilis test utilizing KODE technology Transfusion, Vol 50 (S1), 196A-197A.
Frame et al (2007) Synthetic glycolipid modification of red blood cell membranes Transfusion, Vol 47, 876-882.
Hadac et al (2011) Fluorescein and radiolabelled function-spacer-lipid constructs allow for simple in vitro and in vivo bioimaging of enveloped virions J. Virological Methods, 176, 78-84.
Hammond et al (2007) A gama camera re-evaluation of potassium iodide blocking efficiency in mice Health Phys, Vol. 92, 396-406.
Harrison et al (2010) A synthetic globotriaosylceramide analogue inhibits HIV-1 infection in vitro by two mechanisms Glycobiology, Vol 27, 515-524.
Heathcote et al (2010) Novel antibody screening cells, MUT+Mur kodecytes, created by attaching peptides onto erythrocytes Transfusion, Vol 50, 635-641.
Henry (2009) Modification of red blood cells for laboratory quality control use Curr Opin Hematol, Vol 16, 467-472.
Hermanson (2008) Bioconjugation Techniques, 2nd ed., Academic Press.
Herschman (2003) Molecular imaging: Looking at problems, seeing solutions Science, Vol 302, 605-608.
Kaiser et al (1970) Anal. Biochem., 34(2), 595-8.
Korchagina *et al* (2008) *Fluorescent cell markers* international PCT application no. PCT/NZ2007/000256 (publ. no. WO 2008/030115) .
Kuruppu and Tanabe (2005) Viral oncolysis by herpes simplex virus and other viruses Cancer Biol Ther, Vol 4, 524-531.
Lui et al (2007) Clinical trial results with oncolytic virotherapy: a century of promise, a decade of progress Nat Rev Clin Oncol, Vol 4, 101-117.
Oliver et al (2011) Modeling transfusion reactions and predicting in vivo cell survival with kodecytes Transfusion;(in press).
Peng et al (2003) Hum Gene Ther, Vol, 14, 1565-1577.
Piwnica-Worms et al (2004) Molecular imaging of host-pathogen interactions in intact small animals Cell Microbiol, Vol 6, 319-331.
Raty et al (2006) Magnetic resonance imaging of viral particle biodistribution in vivo Gene Ther, Vol 13, 1440-1446.
Stokke et al (1974) Lipid composition and cholesterol esterification in lymph Scand J Clin Lab Invest, Vol 33, 199-206.
Strable and Finn (2009) Chemical modification of viruses and virus-like particles Curr Top Microbiol Immunol, Vol 327, 1-21.
Tam (1985) A gradative deprotection strategy for the solid phase synthesis of peptide amides using p-(acyloxy)benzhydrylamine resin and the SN2 deprotection method J. Org. Chem., 50, 5291-5298.
Tam and Merrifield (1987) Strong acid deprotection of the synthetic peptides: mechanisms and methods Udenfriend, S., Meienhofer, J., (Eds), The Peptides: Analysis, Synthesis, Biology, Vol. 9, Academic Press, New York, pp 185-248.
Waehler et al (2007) Engineering targeted viral vectors for gene therapy Nat Rev Genet, Vol 8, 573-587.
Yamamoto and Curiel (2010) Current issues and future directions of oncolytic adenoviruses Mol Ther, Vol 18, 243-250.
Yoshimura and Ohnishi (1984) Uncoating of influenza virus in endosomes J Virol, Vol 51, 497-504.

## Claims

1. A **method** of radiolabelling a biological entity comprising the step of contacting a suspension of the biological entity with an iodinated (¹²⁵I) construct of the structure F-S-L for a time and at a temperature sufficient to allow localisation of the ¹²⁵I to the surface of the biological entity where: L is phosphatidylethanolamine; S is a spacer covalently linking F to L and selected to provide a construct that is readily dispersible in water yet spontaneously incorporates into lipid bilayers; and F is a tyrosine residue selected from the group consisting of: 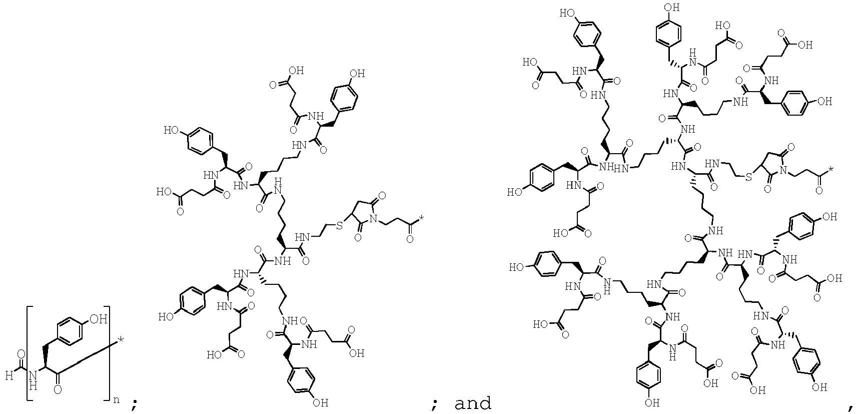 where the biological entity is a cell or a virus and n is an integer from 1 to 6 inclusive and * is other than H.

2. The method of claim 1 where the construct is of the structure: 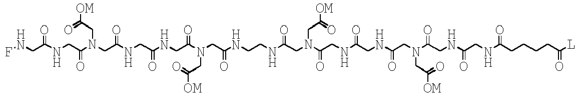 where M is H or CH₃.

3. A non-invasive **method** of monitoring the *in vivo* distribution in a subject of a biological entity producible by the method of claim 1, comprising the step of monitoring the distribution of the ¹²⁵I-labelled biological entity, administered to the subject, by radioactivity bioscanning.

4. The method of claim 3 where the biological entity has been administered to the subject by injection.

5. The method of any one of claims 1 to 4 where the biological entity is a cell or an enveloped virus.

6. A **construct** of the structure: 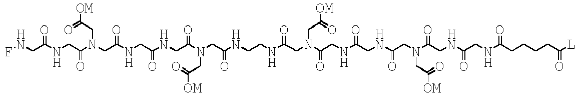 where L is phosphatidylethanolamine, M is H or CH₃, and F is a tyrosine residue selected from the group consisting of: 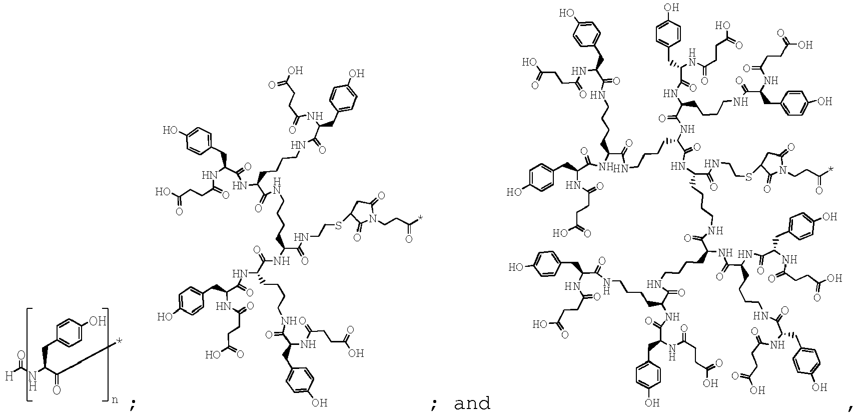 where n is an integer from 1 to 6 inclusive and * is other than H.

7. The construct of claim 6 where L is 1,2-O-dioleoyl-*sn*-glycero-3-phosphatidylethanolamine.

8. The construct of claim 7 where n is 1 and the construct is of the structure: 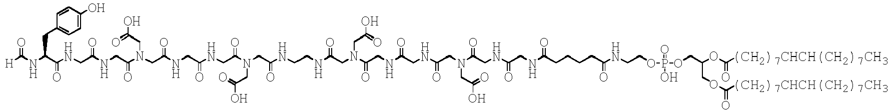 designated FSL-tyrosine.

9. The construct of claim 7 of the structure: 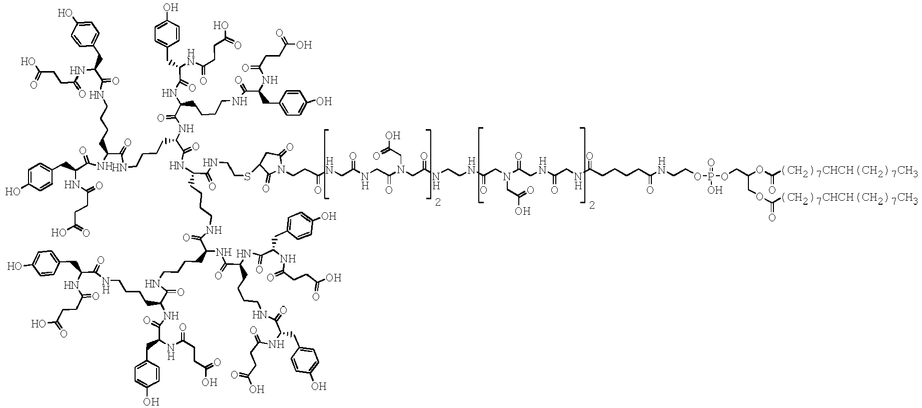 designated FSL-Tyr8.

10. A construct of claim 7 of the structure: 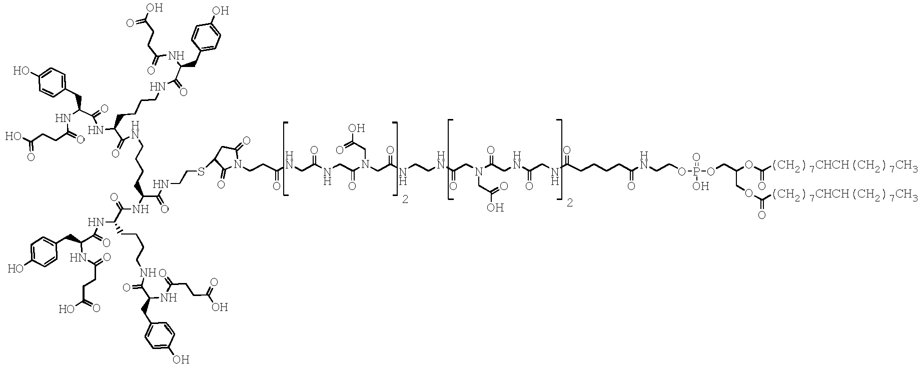 designated FSL-Tyr4

## Patentansprüche

1. Verfahren der Radiomarkierung einer biologischen Einheit, umfassend den Schritt: Kontaktieren einer Suspension der biologischen Einheit mit einem iodierten (¹²⁵I) Konstrukt der Struktur F-S-L über eine ausreichend lange Zeit und bei einer ausreichenden Temperatur für die Lokalisierung des ¹²⁵I auf der Oberfläche der biologischen Einheit, wobei: L Phosphatidylethanolamin ist; S ein Spacer ist, der F kovalent an L bindet und ausgewählt wurde, um ein Konstrukt bereitzustellen, das in Wasser leicht dispergierbar ist, sich sogar spontan in Lipid-Doppelschichten integriert; und F ein Tyrosinrest ist, ausgewählt aus der Gruppe bestehend aus: 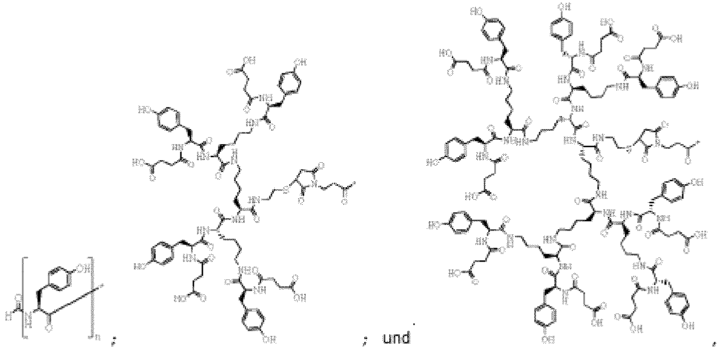 wobei die biologische Einheit eine Zelle oder ein Virus ist und n eine ganze Zahle von 1 bis einschließlich 6 ist und * von H verschieden ist.

2. Verfahren nach Anspruch 1, wobei das Konstrukt folgende Struktur aufweist: 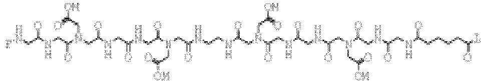 wobei M H oder CH₃ ist.

3. Nichtinvasives Verfahren der Überwachung der in vivo Verteilung in einem Individuum einer biologischen Einheit, die nach dem Verfahren gemäß Anspruch 1 hergestellt werden kann, umfassend den Schritt der Überwachung der Verteilung der ¹²⁵I-markierten biologischen Einheit, die einem Individuum verabreicht wurde, durch Bioscannen mit einem Radioaktivitätsdetektor.

4. Verfahren gemäß Anspruch 3, wobei die biologische Einheit über eine Injektion an ein Individuum verabreicht wurde.

5. Verfahren nach einem beliebigen der Ansprüche 1 bis 4, wobei die biologische Einheit eine Zelle oder ein umhüllter Virus ist.

6. Konstrukt der Struktur: 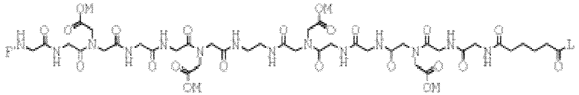 wobei L Phosphatidylethanolamin ist, M H oder CH₃ ist und F ein Tyrosinrest ist, ausgewählt aus der Gruppe bestehend aus: 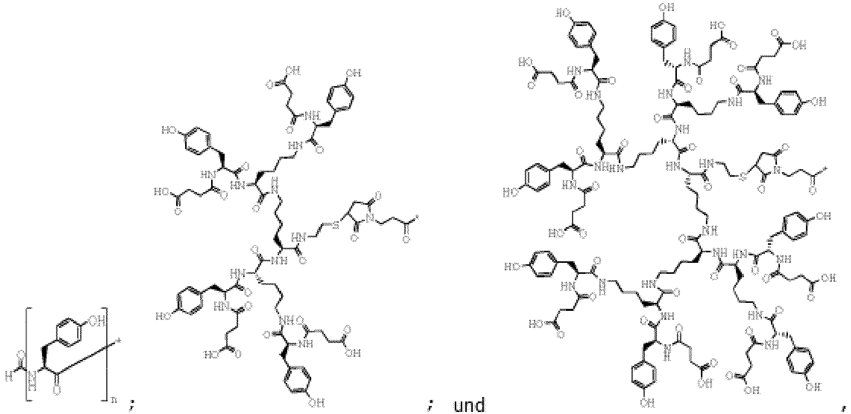 wobei n eine ganze Zahl von 1 bis einschließlich 6 ist und * von H verschieden ist.

7. Konstrukt gemäß Anspruch 6, wobei L 1,2-0-Dioleoyl-sn-glycero-3-phosphatidylethanolamin ist.

8. Konstrukt nach Anspruch 7, wobei n 1 ist und das Konstrukt folgende Struktur aufweist: 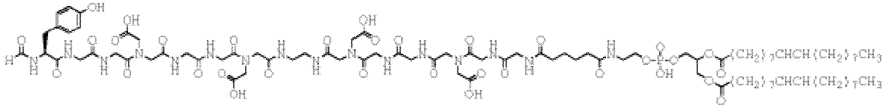 designiertes FSL-Tyrosin

9. Konstrukt gemäß Anspruch 7, mit folgender Struktur: 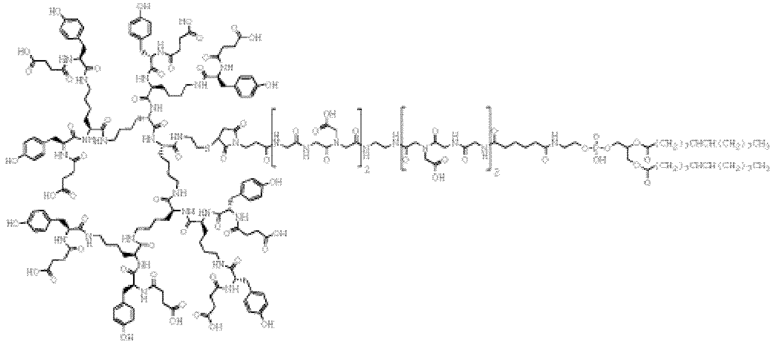 designiertes FSL-Tyr8.

10. Konstrukt gemäß Anspruch 7, mit folgender Struktur: 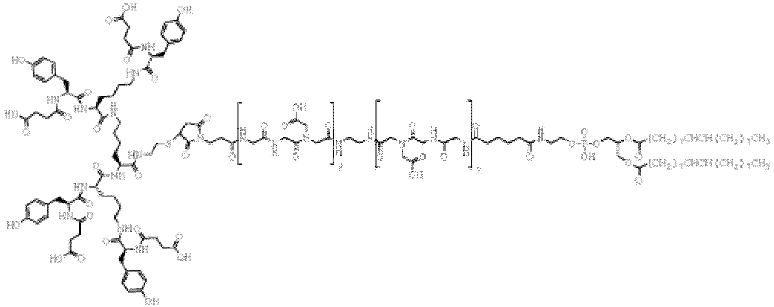 designiertes FSL-Tyr4

## Revendications

1. Procédé de radiomarquage d'une entité biologique comprenant l'étape de mise en contact d'une suspension de l'entité biologique avec une construction iodée (¹²⁵I) de la structure F-S-L pendant une durée et à une température suffisante pour permettre la localisation de ¹²⁵I au niveau de la surface de l'entité biologique, où :
L est la phosphatidyléthanolamine ; S est un espaceur liant de façon covalente F à L et choisi pour fournir une construction qui est facilement dispersible dans l'eau tout en s'incorporant spontanément à des bicouches lipidiques ;
et F est un résidu de tyrosine choisi dans le groupe consistant en : 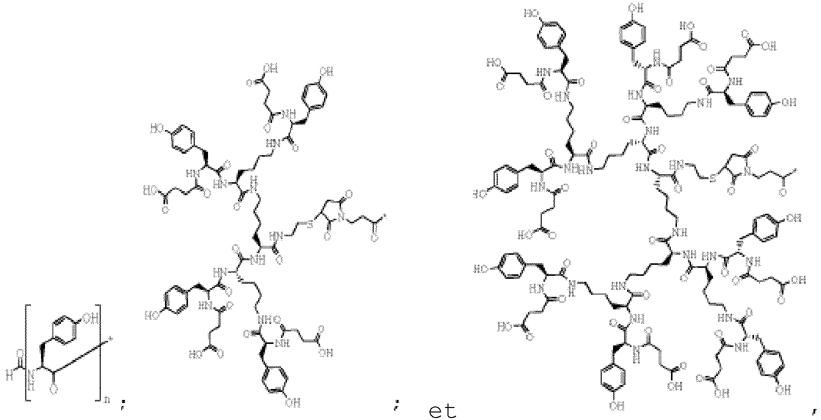 où l'entité biologique est une cellule ou un virus et n est un nombre entier de 1 à 6 inclus et * est autre que H.

2. Procédé selon la revendication 1, dans lequel la construction a la structure : 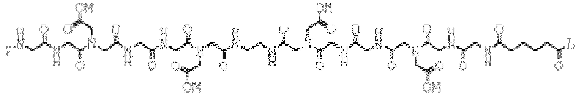 où M est H ou CH₃.

3. Procédé non invasif de surveillance de la répartition *in vivo* chez un sujet d'une entité biologique pouvant être produite par le procédé selon la revendication 1, comprenant l'étape de surveillance de la répartition de l'entité biologique marquée par ¹²⁵I, administrée au sujet, par bio-balayage de radioactivité.

4. Procédé selon la revendication 3, dans lequel l'entité biologique a été administrée au sujet par injection.

5. Procédé selon l'une quelconque des revendications 1 à 4, dans lequel l'entité biologique est une cellule ou un virus enveloppé.

6. Construction de la structure : 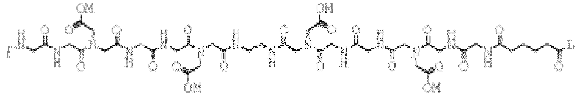 où L est la phosphatidyléthanolamine, M est H ou CH₃, et F est un résidu de tyrosine choisi dans le groupe consistant en : 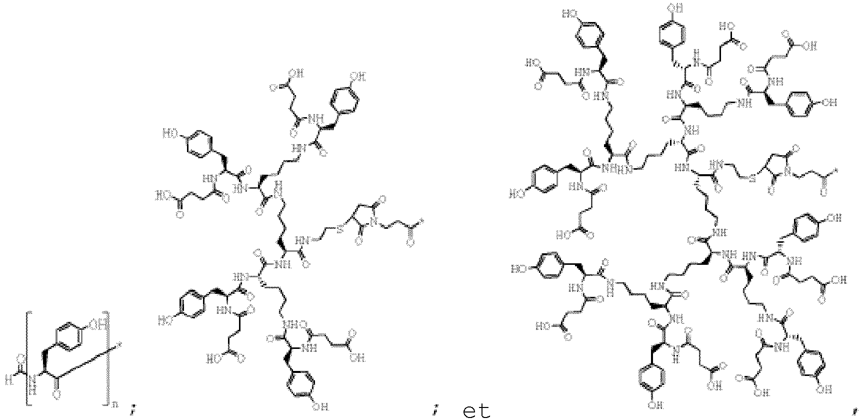 où n est un nombre entier de 1 à 6 inclus et * est autre que H.

7. Construction selon la revendication 6, dans laquelle L est la 1,2-O-dioléoyl-*sn*-glycéro-3-phosphatidyléthanolamine.

8. Construction selon la revendication 7, dans laquelle n est 1 et la construction a la structure : 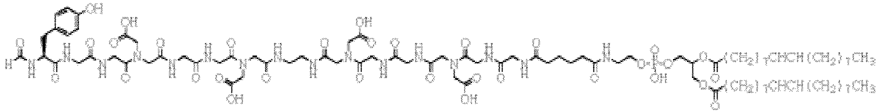 appelée FSL-tyrosine.

9. Construction selon la revendication 7, ayant la structure : 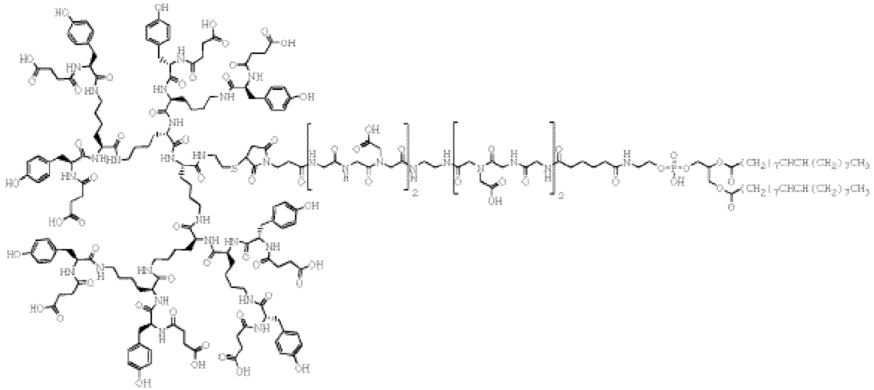 appelée FSL-Tyr8.

10. Construction selon la revendication 7, ayant la structure : 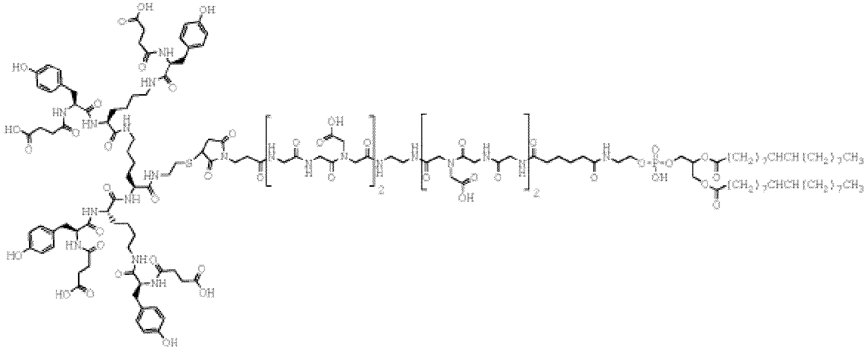 appelée FSL-Tyr4.
